# EUROPEAN PATENT SPECIFICATION

(11) **EP 1 769 021 B1**
(45) Date of publication and mention of the grant of the patent: **12.08.2009**
(21) Application number: 05756648.1
(22) Date of filing: 20.05.2005
(51) Int. Cl.: C08J 5/18, C08L 67/02, A61L 15/22, C08L 3/00

(54) **USE OF PERFORATED BIODEGRADABLE FILMS AND SANITARY PRODUCTS OBTAINED THEREFROM**
VERWENDUNG VON PERFORIERTEN BIOLOGISCH ABBAUBAREN FOLIEN UND DARAUS ERHALTENE HYGIENEPRODUKTE
UTILISATION DES PELLICULES BIODEGRADABLES PERFOREES ET PRODUITS SANITAIRES OBTENUS A PARTIR DE CES PELLICULES

(30) Priority: 25.05.2004 IT MI20041039
(43) Date of publication of application: 04.04.2007
(73) Proprietor: NOVAMONT S.p.A., 28100 Novara (IT)
(72) Inventor: DEL TREDICI, Gianfranco, I-21018 Sesto Calende (VA) (IT)
(74) Representative: Zanoli, Enrico
(86) International application number: PCT/EP2005/005495
(87) International publication number: WO 2005/116118

(56) References cited:
- EP-A- 1 116 748
- WO-A-03/089490
- DE-A1- 10 054 650
- US-A- 5 417 679

## Description

The present invention relates to the use of perforated biodegradable films for sanitary products , in particular as topsheets for sanitary articles for women.

The films used as internal films in contact with the skin (referred to as topsheets) for sanitary articles for women are currently for the most part produced with synthetic materials, typically polyethylene or non-woven fabric made of polypropylene or polyethylene coupled to non-woven fabric. Consequently, they are products of a completely synthetic origin and may therefore constitute a possible source of low compatibility with the skin with which they come into contact.

DE 100 54 650 A1 discloses compositions for backsheets of sanitary articles and mentions the possibility to use blends of partially aromatic polyesters with starch to make backsheets for sanitary articles. It also mentions the possibility to make perforations in backsheets containing generic fillers. However, it does not disclose a topsheet for sanitary articles, namely a sheet designed to be in contact with the skin of a user.

US 5,417,679 discloses biodegradable films for absorbent articles without perforations for use as backsheets for sanitary articles. When topsheets are mentioned (Ex. III), a conventional structure made of polypropylene fibres is described.

A drawback of a more general nature linked to the use of current materials depends upon their origin from non-renewable sources.

A further drawback is their high environmental impact since they cannot be disposed of along with the fraction of waste to be sent on for composting but must be disposed of in dumps or by incineration.

The aforementioned drawbacks are overcome by using as topsheet for sanitary articles the perforated biodegradable film according to the present invention, which proves particularly suited for being used as topsheet for sanitary articles for

The perforated film as topsheet according to the present invention can however be conveniently used for further applications also in fields different from the sanitary field.

By way of non-limiting example, it may be mentioned the use of the film as absorbent material for packaging foodstuffs, such as meat and fish, which may present leakage of liquid.

The perforated biodegradable film as topsheet according to the invention has a greater biocompatibility than traditional plastic materials. The film moreover exhibits mechanical and functional properties comparable with those of plastic films of synthetic origin. In particular, the film has an excellent wetback property (i.e., the capacity for the perforated film not to allow fluids to flow back even under pressure to the area of the body from which they come) and an excellent strike-through time (i.e., the time required for a liquid to pass through the film), which render it particularly suited for receiving body fluids.

Finally, the perforated film exhibits excellent characteristics of flexibility, i.e., it is readily conformable to the body surfaces and responds promptly to the external forces of deformation. In particular, the present invention is aimed at the use as topsheet of a film produced from a composition containing at least one aliphatic or aliphatic-aromatic biodegradable polymer, from dicarboxylic acid, and from a diol in a mixture with at least one polysaccharide derivative.

Examples of diacids are succinic acid, oxalic acid, malonic acid, glutaric acid, adipic acid, pimelic acid, suberic acid, undecanoic acid, dodecanoic acid, azelaic acid, sebacic acid, and brassilic acid. Particularly preferred are azelaic acid, sebacic acid, and brassilic acid or mixtures thereof.

Specific glycols are ethylene glycol, diethylene glycol, triethylene glycol, polyethylene glycol, 1,2- and 1,3-propylene glycol, dipropylene glycol, 1,3-butane diol, 1,4-butane diol, 3-methyl-1,5-pentane diol, 1,6-hexane diol, 1,9-nonane diol, 1,11-undecane diol, 1,13-tridecane diol, neopentyl glycol, polytetramethylene glycol, 1,4-cyclohexane dimethanol and cyclohexane diol. These compounds can be used alone or in mixtures. Particularly preferred are ethylene glycol, diethylene glycol and 1,4-butane diol.

As regards the aromatic part, the biodegradable polymer used in the perforated film according to the present invention preferably contains a polyfunctional aromatic compound such as, for example, a phthalic acid, in particular terephthalic acid, bisphenol A, hydroquinone, and the like. Terephthalic acid is particularly preferred.

Particularly preferred are polymers with an aromatic part constituted by terephthalic acid and an aliphatic part constituted by diacid diols, with C₂-C₂₀ aliphatic chain, whether branched or otherwise (possibly with a chain extended with isocyanates, anhydrides or epoxides) and in particular polyesters with a base of terephthalic acid, adipic acid or sebacic acid and butane diol. Examples of this type of materials are the product sold with the trade name Ecoflex by BASF AG or the product sold with the trade name Eastarbio by Eastman Chemical.

The biodegradable polyesters used for the production of the film according to the invention can be polymerized via polycondensation or, as in the case of glycolide and of lactones, by ring opening, as is known in the literature. In addition, the polyesters can be polymers branched through the introduction of polyfunctional monomers such as glycerine, epoxidized soya oil, and trimethylol propane or polycarboxylic acids such as butantetracarboxylic acid. Furthermore, the polyesters may also be modified with chain extensors such as difunctional, trifunctional or tetrafunctional anhydrides, such as maleic anhydride, trimellitic anhydride and pyromellitic anhydride, with polyepoxides, or with aliphatic and aromatic isocyanates.

Regrading with isocyanate can occur in the molten state, either at the end of the reaction of polymerization or in the step of extrusion, or in the solid state.

In order to improve the characteristics of filmability, there may be added amides of aliphatic acids, such as oleamide, stearamide, erucamide, behenamide, N-oleylpalmitamide, N-stearylerucamide and other amides, salts of fatty acids, such as aluminium stearate, zinc stearate or calcium stearate, and the like. The amounts of these additives may vary from 0.05 to 7 parts and preferably between 0.1 and 5 parts on the mixture of polymers.

The polysaccharide used for preparation of the mixture for the production of the film according to the present invention can be a native starch, such as preferably starches from maize, potatoes, tapioca, rice, wheat, peas and even a starch with high contents of amylose, and the so-called waxy starches. Physically and/or chemically modified starches can also be used, including ethoxylated starches, oxypropylated starches, acetated starches, butyrated starches, propinated starches, cationic starches, oxidized starches, reticulated starches, gelatinized starches, destructured starches and starches complexated by polymeric structures.

Destructurized native maize starch is particularly preferred.

Conveniently, the mixture for the production of the film according to the invention can contain one or more plasticizers. The plasticizers that can be used are, for example, the ones described in the patent EP-0 575 349, the content of which is incorporated in the present invention. Particularly suitable are glycerine, sorbitol, mannitol, erythritol, polyvinyl alcohol with low molecular weight, in addition to the oxyethylated and oxypropylated derivatives of the aforesaid compounds, citrates and acetines.

The starting compositions can moreover contain suitable additives, such as lubricating agents or dispersing agents, colouring agents, fillers, surfactants.

With reference to the surfactants, non ionic surfactants, such as low molecular PEG, are preferred. They can be directly incorporated in the composition or coated on the film according to the invention.

The starting compositions for the production of the film according to the invention can be fed directly to the extruder or else can be fed in the form of pre-formed granules.

Perforated films are already used in pantie liners; in particular, wide use is made of plastic films perforated in vacuum conditions with funnel-shaped holes. As regards the technology of production, there exist different methods of perforation, such as for example:
(1) hot etching;
(2) calendering by friction between two cylinders one of which is engraved and the other smooth;
(3) cutting and extension perpendicular to the cut;
(4) use of male-female cylinders;
(5) hot-needle method; and
(6) use of jets of water on a mesh support or on a perforated drum to guarantee formation of through holes.

For the perforated biodegradable films according to the invention hot perforation in vacuum conditions with production of conical holes is particularly preferred. Particularly advantageous is the use of holes with different shapes thus allowing to increase the resistance to the elastic return of the film. Particularly preferred are perforated biodegradable films with an open area (i.e. the overall area of the holes) of between 5% and 75%, preferably between 10% and 65%, and still more preferably between 15% and 55%, with a hole density between 10 and 200 holes/cm², preferably between 30 and 150 holes/cm² and still more preferably between 50 and 120 holes/cm².

The biodegradable perforated film according to the present invention can be advantageously used also for providing a completely biodegradable sanitary article when associated to other components made of biodegradable material.

In particular, the biodegradable perforated film according to the present invention can be advantageously combined with a biodegradable film used as backsheet (i.e., the film set on the outer surface of the hygiene article with containment functions) for sanitary articles. In a particularly advantageous way, said backsheet is constituted by a biodegradable film having a starch-based composition.

An important function that must be performed by the film used as backsheet in sanitary products such as disposable nappies or pantie liners is that of biological barrier, i.e., the property of the film to block passage of possible pathogenic agents from the part in contact with the body towards the outer environment, and viceversa. Another important property of the film used as backsheet in sanitary products is breathability, i.e., the ability of the film to be impermeable to liquids and permeable to vapours.

Currently, the breathable film set on the outer surface (backsheet) of most sanitary products is made of microperforated polyethylene. The breathable film made of polyethylene is provided with microholes that increase the permeability to water vapour considerably, thus facilitating transpiration. However, the presence of the microholes represents a limit to the actual barrier that the film can exert. The backsheet in fact is not capable of blocking passage of possible pathogenic agents present in the faeces outwards, i.e., it is not able to act as a complete biological barrier.

The possibility of a contamination of the backsheet caused by agents present in the faeces may have severe consequences from the hygienic-sanitary standpoint, especially in communities where there are small children (nurseries, infant schools, hospitals, etc.).

Starch-based biodegradable materials are characterized by high values of permeability to water vapour. Such values are sufficiently high to enable their use as backsheet films. Said permeability is not due to the presence of microholes in the structure of the film, but to the starch, which has a high permeability to water vapour. Tests carried out according to the ASTM F 1671-97b standard on films made of Mater-Bi^{®} NF01U, a material produced by Novamont SpA, have shown that said films act as a complete biological barrier. The same tests carried out on microperforated polyethylene indicate that this material enables passage of viruses from one side of the film to the other. Consequently, microperforated polyethylene affords advantages from the standpoint of comfort, but proves inadequate from the sanitary standpoint.

Finally, the perforated biodegradable film according to the invention exhibits excellent processability and a complete compatibility with the new types of non-woven fabric.

### EXAMPLE 1

A film made with a composition with a base of starch and aliphatic aromatic polyester commercially available from Novamont SpA under the trademark Mater-Bi^{®} NF866, was fed into a machine for hot embossing in vacuum conditions with funnel-shaped holes to obtain a film having a substance (mass per unit area) of approximately 25 g/m² and conical perforations. The density of the holes was approximately 100 holes/cm².

Said film was then tested to evaluate the level of cytotoxicity thereof as well as the mechanical and functional properties.

### Cytotoxicity test

A film having a composition according to the invention but without perforations was compared with a film of polyethylene coupled to non-woven fabric commonly used as backsheet in hygiene articles. The films were analysed according to the methods listed below.
1) Quantitative assessment of the cell growth by means of measurement of the number of dead cells via counting in a Burker chamber.
2) Highlighting of any possible morphological alterations, such as, alteration of the cell "spreading" (flattened shape), presence of vacuoles (universally considered as a symptom of cellular suffering), and presence of granules released by the cells in the culture medium.
3) Evaluation of cell vitality by means of the method of exclusion of Trypan Blue (vital stain) and control at the optical microscope in phase contrast.

The tests were carried out on a cell line of keratinocytes (HaCat), i.e. epidermal cells that constitute the outermost skin layer. The cells were spread in 6-well dishes and cultured in complete DMEM medium in bovine foetal serum and antibiotics in a thermostatted environment at 37°C in an atmosphere of CO₂ at 95% and O₂ at 5%. The experiments were conducted starting from a total of 500,000 cells per specimen and setting them in contact with 1 cm² of film reduced into 10 fragments of equal dimensions.

Cells in a culture without the addition of film were used as control. The qualitative and quantitative evaluations were made after 24 hours of incubation. Six experiments were carried out.

The results of the test are listed hereinafter.
1) The cell growth was significantly greater in the presence of the Mater-Bi NF866 film as compared to the case where the film of coupled polyethylene was used, whilst the difference between the case where the NF866 film was used and the control was not significant, this confirming that the Mater-Bi NF866 film does not have a cytotoxic activity.
   The cells in contact with the film made of coupled polyethylene had a growth significantly smaller than that of the cells treated with the Mater-Bi NF866 film and than that of the control.
2) Evaluation of the morphology did not reveal any cell alteration.

The perforated biodegradable films according to the present invention consequently show characteristics of non-cytotoxicity, which highlight the biocompatibility thereof, in particular a biocompatibility that is higher than that of the film made of polyethylene. The film according to the invention has been tested with respect to its functional and mechanical properties. The results are reported in the herebelow table in comparison with a polyethylene film. In the table are also listed figures relating to a film according to the invention which has been coated with 0.5% by weight of a surfactant consisting of low molecular polyethylenglycol (Mw < 600).

### Mechanical and functional tests

| | Unit | Method | Polyethylene | NF 866 | Mater-Bi® NF806 + surfactant |
|---|---|---|---|---|---|
| Grammage | g/m² | ASTM D3776 | 22 | 25.6 | 27 |
| Ultimate tensile strength | N/m² | ASTM D 882 | 14 | 10 | 15 |
| Ultimate elongation MD | % | ASTM D 882 | 85 | 103 | 130 |
| Stress to produce 5% elongation MD | N/m² | ASTM D 882 | 4.20 | 3.5 | 3.9 |
| Wetback | g | EDANA 151.3 | 0.06 | 0.06 | 0.09 |
| Strike-through time | s | EDANA 150.5 | 2.9 | 3.5 | 2.7 |
| Open area | % | TFPI | 23 | 15 | 34 |
| Run off | g | EDANA 152.1-02 | 3.1 | 1.1 | 0 |

## Claims

1. Use of a perforated biodegradable film as topsheet for sanitary articles, said film comprising:
- at least one aliphatic or aliphatic-aromatic biodegradable polyester obtained from a dicarboxylic and a diol;
- at least one polysaccharide.

2. Use of a perforated biodegradable film according to claim 1, wherein said polysaccharide is starch.

3. Use of a perforated biodegradable film according to claim 2, wherein said starch is maize starch.

4. Use of a perforated biodegradable film according to claim 1, wherein said biodegradable polyester is an aliphatic-aromatic polyester of the dicarboxylic acid -diol type.

5. Use of a perforated biodegradable film according to claim 4, wherein said aliphatic-aromatic polyester is linear or branched co-terephthalate polybutylene adipate.

6. Use of a perforated biodegradable film according to any of the preceding claims, **characterised by** an open area comprised between 5% and 75%.

7. Use of a perforated biodegradable film according to claim 6, **characterised by** an open area comprised between 10% and 65%.

8. Use of a perforated biodegradable film according to claim 7, **characterised by** an open area comprised between 15% and 55%.

9. Use of a perforated biodegradable film according to any of the claims from 1 to 5, **characterised by** a hole density comprised between 10 and 200 holes/cm².

10. Use of a perforated biodegradable film according to claim 9, **characterised by** a hole density comprised between 30 and 150 holes/cm².

11. Use of a perforated biodegradable film according to claim 10, **characterised by** a hole density comprised between 50 and 120 holes/cm².

12. Use of a perforated biodegradable film according to claims from 6 to 11, wherein the perforation is a hot perforation performed under vacuum conditions with production of conical holes.

13. Sanitary article comprising the perforated biodegradable film according to any of the preceding claims, wherein said biodegradable film is an internal film or topsheet intended to be in contact with the skin of a user.

14. Sanitary article according to claim 13, wherein said biodegradable film is an internal film or topsheet, **characterised by** further comprising a breathable and complete-biological-barrier film made of a biodegradable material as outer film or backsheet.

15. Sanitary article according to claim 14, wherein said breathable and complete-biological-barrier film made of a biodegradable material is a starch-based film.

## Patentansprüche

1. Verwendung einer perforierten biologisch abbaubaren Folie als Decklage für Sanitärartikel, wobei die Folie umfasst:
- mindestens einen aus einer Dicarbonsäure und einem Diol erhaltenen aliphatischen oder aliphatischaromatischen biologisch abbaubaren Polyester; und
- mindestens ein Polysaccharid.

2. Verwendung einer perforierten biologisch abbaubaren Folie gemäß Anspruch 1, worin das Polysaccharid Stärke ist.

3. Verwendung einer perforierten biologisch abbaubaren Folie gemäß Anspruch 2, worin die Stärke Maisstärke ist.

4. Verwendung einer perforierten biologisch abbaubaren Folie gemäß Anspruch 1, worin der biologisch abbaubare Polyester ein aliphatisch-aromatischer Polyester vom Dicarbonsäure-Dioltyp ist.

5. Verwendung einer perforierten biologisch abbaubaren Folie gemäß Anspruch 4, worin der aliphatischaromatische Polyester lineares oder verzweigtes Co-Terephthalatpolybutylenadipat ist.

6. Verwendung einer perforierten biologisch abbaubaren Folie gemäß mindestens einem der vorhergehenden Ansprüche, **gekennzeichnet durch** eine offene Fläche zwischen 5 und 75 %.

7. Verwendung einer perforierten biologisch abbaubaren Folie gemäß Anspruch 6, **gekennzeichnet durch** eine offene Fläche zwischen 10 und 65 %.

8. Verwendung einer perforierten biologisch abbaubaren Folie gemäß Anspruch 7, **gekennzeichnet durch** eine offene Fläche zwischen 15 und 55 %.

9. Verwendung einer perforierten biologisch abbaubaren Folie gemäß mindestens einem der Ansprüche 1 bis 5, **gekennzeichnet durch** eine Loch-Dichte zwischen 10 und 200 Löchern/cm².

10. Verwendung einer perforierten biologisch abbaubaren Folie gemäß Anspruch 9, **gekennzeichnet durch** eine Loch-Dichte zwischen 30 und 150 Löchern/cm².

11. Verwendung einer perforierten biologisch abbaubaren Folie gemäß Anspruch 10, **gekennzeichnet durch** eine Loch-Dichte zwischen 50 und 120 Löchern/cm².

12. Verwendung einer perforierten biologisch abbaubaren Folie gemäß Anspruch 6 bis 11, worin die Perforierung eine unter Vakuumbedingungen durchgeführte Heißperforierung mit Erzeugung von konischen Löchern ist.

13. Sanitärartikel, der die perforierte biologisch abbaubare Folie gemäß mindestens einem der vorhergehenden Ansprüche umfasst, worin die biologisch abbaubare Folie eine innere Folie oder eine für den Kontakt mit der Haut des Benutzers vorgesehene Deckfolie ist.

14. Sanitärartikel gemäß Anspruch 13, worin die biologisch abbaubare Folie eine innere Folie oder Decklage ist, **dadurch gekennzeichnet, dass** er weiterhin eine atmungsaktive und vollständige biologische Barrierenfolie aus einem biologisch abbaubaren Material als äußere Folie oder Rückseitenlage umfasst.

15. Hygieneartikel gemäß Anspruch 14, worin die atmungsaktive und vollständige biologische Barrierenfolie auf biologisch abbaubarem Material eine Folie auf Stärkebasis ist.

## Revendications

1. Utilisation d'un film biodégradable perforé comme revêtement de surface pour des articles sanitaires, ledit film comprenant :
- au moins un polyester biodégradable aliphatique ou aliphatique-aromatique, obtenu à partir d'un acide dicarboxylique et d'un diol ;
- au moins un polysaccharide.

2. Utilisation d'un film biodégradable perforé selon la revendication 1, dans lequel ledit polysaccharide est l'amidon.

3. Utilisation d'un film biodégradable perforé selon la revendication 2, dans lequel ledit amidon est de l'amidon de maïs.

4. Utilisation d'un film biodégradable perforé selon la revendication 1, dans lequel ledit polyester biodégradable est un polyester aliphatique-aromatique du type acide dicarboxylique-diol.

5. Utilisation d'un film biodégradable perforé selon la revendication 4, dans lequel ledit polyester aliphatique-aromatique est un poly(butylène adipate-co-téréphtalate) linéaire ou ramifié.

6. Utilisation d'un film biodégradable perforé selon l'une quelconque des revendications précédentes, **caractérisé par** une surface ouverte comprise entre 5 % et 75%.

7. Utilisation d'un film biodégradable perforé selon la revendication 6, **caractérisé par** une surface ouverte comprise entre 10 % et 65 %.

8. Utilisation d'un film biodégradable perforé selon la revendication 7, **caractérisé par** une surface ouverte comprise entre 15 % et 55 %.

9. Utilisation d'un film biodégradable perforé selon l'une quelconque des revendications 1 à 5, **caractérisé par** une densité de perforation comprise entre 10 et 200 perforations/cm².

10. Utilisation d'un film biodégradable perforé selon la revendication 9, **caractérisé par** une densité de perforation comprise entre 30 et 150 perforations/cm².

11. Utilisation d'un film biodégradable perforé selon la revendication 10, **caractérisé par** une densité de perforation comprise entre 50 et 120 perforations/cm².

12. Utilisation d'un film biodégradable perforé selon les revendications 6 à 11, dans lequel la perforation est une perforation à chaud réalisée dans des conditions sous vide, ce qui entraîne la formation de perforations coniques.

13. Article sanitaire comprenant le film biodégradable perforé selon l'une quelconque des revendications précédentes, dans lequel ledit film biodégradable est un film intérieur ou un revêtement extérieur censé être en contact avec la peau d'un utilisateur.

14. Article sanitaire selon la revendication 13, dans lequel ledit film biodégradable est un film intérieur ou un revêtement extérieur, **caractérisé en ce qu'**il comprend, en outre, un film de barrière biologique complète, respirant, constitué d'un matériau biodégradable, comme film externe ou comme feuille de fond.

15. Article sanitaire selon la revendication 14, dans lequel ledit film de barrière biologique complète, respirant et constitué d'un matériau biodégradable, est un film à base d'amidon.
